# EUROPEAN PATENT SPECIFICATION

(11) **EP 3 266 447 B1**
(45) Date of publication and mention of the grant of the patent: **03.03.2021**
(21) Application number: 17000116.8
(22) Date of filing: 24.01.2017
(51) Int. Cl.: A61K 9/16, A61K 9/20, A61K 31/519

(54) **PRODUCTION PROCESS FOR FORMULATIONS CONTAINING TICAGRELOR**
HERSTELLUNGSVERFAHREN FÜR FORMULIERUNGEN MIT TICAGRELOR
PROCÉDÉ DE PRODUCTION DE FORMULATIONS CONTENANT DU TICAGRELOR

(43) Date of publication of application: 10.01.2018
(73) Proprietor: Ali Raif Ilac San. A.S., 34418 Kagithane, Istanbul (TR)
(72) Inventor: Unlu, Serdar, Basaksehir Istanbul (TR); Isik, Murat Muhibbi, Basaksehir Istanbul (TR); Akcin, Selin, Basaksehir Istanbul (TR); Demir, Filiz, Basaksehir Istanbul (TR); Demir, Sevda, Basaksehir Istanbul (TR); Malkoc, Mehmet, Basaksehir Istanbul (TR)
(74) Representative: Bulut, Pinar

(56) References cited:
- WO-A1-2014/006091
- WO-A1-2014/059955
- WO-A1-2014/083139
- WO-A1-2014/170026
- WO-A2-2011/076749
- WO-A2-2013/150495
- WO-A2-2014/118808
- "Amorphous Forms of (1S,2S,3R,5S)-3-[7-[[(1R,2S)-2-(3,4-difluo rophenyl)cyclopropyl] amino]-5-(propylthio)-3H-1,2,3-triazolo[4, 5,d]pyrimidine-3-yl]-5-(2- hydroxyethoxy)-1,2-cyclopentanediol", IP.COM JOURNAL, IP.COM INC., WEST HENRIETTA, NY, US, 15 June 2011 (2011-06-15), XP013145347, ISSN: 1533-0001

## Description

### Technical field of invention

This present invention is related to production process of formulations containing ticagrelor and use of these formulations in the prevention of thrombotic events in patients with acute coronery syndrome.

### State of the art

Thrombocyte activation is one of the main steps in development of acute coronary syndrome (ACS). For this reason, inhibition of thrombocyte activation is one of the most important points of the treatment. There exists three aims for inhibition of thrombocyte activation. The first one is the inhibition of cyclooxygenase, the second one is inhibition of adenosine 5-diphosphate (ADP) (by P2Y12 receptor inhibition) and the third one is inhibition of glycoprotein IIb/IIa receptor.

One of the components that act by inhibition of adenosine 5-diphosphate is ticagrelor. Ticagrelor is a reversible, selective P2Y12-receptor antagonist. Its chemical name is (1S,2S,3R,5S)-3-(7-{[(1R,2S)-2-(3,4-Diflourophenyl)cyclopropyl]amino}-5-(propylsulfanyl)-3H-[1,2,3]triazol[4,5-d]pyrimidine-3-yl)-5-(2-hydroxyethoxy)cyclopentane-1,2-diol and its molecular structure is given in figure 1. Ticagrelor is first described in patent number WO9905143 and described more specifically in patent number WO0034283.

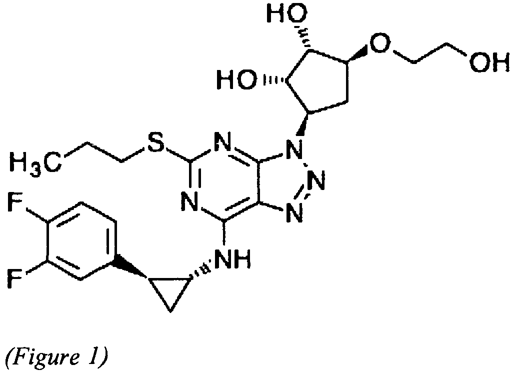

Product containing ticagrelor is marketed by AstraZeneca under the tradename of Brilique and/or Brilinta in the dosage form of 60 mg and 90 mg oral tablet.

### The technical problems expected to be solved by the invention

One of criteria that pharmaceutical formulations with oral use should hold is appropriate bioavailability. Bioavailability represents the ratio of the active ingredient that enters the systemic circulation. Ticagrelor is administered by oral route with a relatively low bioavilability of 36%.

One of the most important factors that affects the bioavailability is the release of the active ingredient from the final dosage form. For this reason, release of the entire amount of the active ingredient from the final dosage form is important. Release of the active ingredient from the final dosage form is variable depending on the physical properties of the active ingredient besides the release profile of the dosage form. One of the most important physical properties having important role in the release ratio is the solubility of the active ingredient.

Ticagrelor has a very low solubility in water which is around 10 µg/mL. Besides, it also has a low permeability. Due to this, it is classified in the class 4 compounds which have low solubility and low permeability within the Biopharmaceutical Classification System.

There are studies reported that aimed to improve the solubility of active ingredient ticagrelor. EP2515871 B1 patent reported the development of solid dosage forms of tikagelor active ingredient with D90 value in the range of 1-150 µm and this dosage form is stated to have the desired solubility and bioavailability levels.

Grinding technique is used to obtain ticagrelor active ingredient with the desired particule size according to the above mentioned patent document. Larger surface area provided by minimization of the particule size improves the solubility and the content uniformity in the final dosage form.

However, physical processes applied to the active ingredient, like grinding, may result in the degradation of the active ingredient. Besides, such processes have been observed to result in the change of polymorphic form of the active ingredient, especially causing tendency in the change of crystal form of active ingredient into amorph form. Amorph forms are less preferred in pharmaceutical formulations as they are more hygroscopic and less stable when compared to the crystal forms.

In addition, it is known that minimization of the particule size leads to deterioration in the flow properties of the active ingredient. Active ingredient particules with poor flow properties leads to difficulties in production, non-uniformity in the final dosage form and poor solubility profile.

Finally, adjusting the particule size within a specific range after the synthesis of active ingredient extends the production process, requires additional equipment and increases the cost.

In order to overcome the problems caused by the particule size adjustment before formulation of the active ingredient in the prior art, development of formulation production methods that can be applied independent of the active ingredient particule size are required.

### Description of the invention

Inventors have surprisingly achieved to formulate ticagrelor active ingredient independent of the particule size and so they have overcomed the insufficient solubility and content uniformity problems that are faced when particule size are not adjusted in the prior art.

In this production method, adjusting the particule size of ticagrelor active ingredient is not necessary. Hence, this process can be applied to ticagrelor active ingredient with a wide range of particule size and as a result, formulations with similar solubility profile and similar content uniformity can be produced.

In this particule size independent production method, ticagrelor active ingredient is dissolved and/or dispersed in a solvent. This solvent can be an inorganic or organic solvent or a mixtures thereof in which ticagrelor active ingredient can dissolve and/or dispersed.

In this production method, the solvent in which ticagrelor active ingredient is dissolved and/or dispersed can be methanol, ethanol, n-propanol, isopropanol, n-butanol, isobutanol, dichlomethane, butanone, acetone, dimethyl sulfoxide, glycerine, propylene glycol, glycol, water or mixture of these with each other and/or any pharmaceutically acceptable organic and/or inorganic solvent.

Above mentioned solvent mixtures can be formed by mixture of two or more organic or inorganic solvents and there is no limitation in the number of solvents.

Although solvent mixtures are not limited with the below mentioned ones, a solvent mixture can be selected among methanol/water, methanol/isopropanol, methanol/dicholomethane, methanol/dicholomethane/isopropanol, methanol/dicholomethane/water, ethanol/water, ethanol/isopropanol, ethanol/dicholomethane, ethanol/dicholomethane/water, ethanol/ dicholomethane/isopropanol, ethanol/dicholomethane/isopropanol/water, propylene glycol/water, propylene glycol/ethanol, propylene glycol/water/ethanol.

Amount of every solvent in this solvent mixture can be 0.1 to 99.9% by weight. For instance, weight percent ratio of solvents can be in the 99.9:0.1% and 0.1:99.9% range, 99.8:0.1:0.1% and 0.1:99.8:0.1% and 0.1:0.1:99.8% range.

In this production method according to the invention, ticagrelor active ingredient is mixed with other excipients of the formulation after it is dissolved and/or dispersed in a solvent.

In one aspect, this production method basically involves wet granulation method. In this production method ticagrelor active ingredient is dissolved and/or dispersed in granulation solvent/solvent mixture.

Wet granulation method involves wet granulators such as mixer granulator, high shear granulator, fluidized bed granulator which are commonly used in pharmaceutical industry.

The subject of production method according to the invention basically involves the below steps;
a. preparation of the granulation solution by dissolving and/or dispersing ticagrelor active ingredient in a proper solvent/solvent mixture,
b. dry mixing of one or more excipients,
c. granulation of the dry mixture obtained at step b with the granulation solution containing ticagrelor obtained at step a,
d. drying and sieving the granules obtained at step c and production of the desired dosage form.

More specifically the subject of production method according to the invention involves the below steps;
a. preperation of the granulation solution by dissolving and/or dispersing ticagrelor active ingredient in a suitable solvent/solvent mixture,
b. dry mixing of one or more excipients,
c. granulation of the dry mixture obtained at step b with the granultaion solution with ticagrelor obtained at step a,
d. drying and sieving the granules obtained at step c,
e. re-granulation of the granules obtained at step d with a granulation solvent/solvent mixture different from that used in step a,
f. drying and sieving of the granules obtained at step e and production of the desired dosage form.

The present formulation according to the invention contains 10-50%, preferably 20-40% ticagrelor or its pharmaceutically acceptable salt.

Accroding to the different aspect, the formulation according to the invention contains 30-360 mg of ticagrelor or any pharmaceutically acceptable salt of ticagrelor in an equivalent amount. The formulation according to the invention preferably contains 60-120 mg of ticagrelor or any pharmaceutically acceptable salt of ticagrelor in an equivalent amount.

Ticagrelor active ingredient used in the present formulation according to the invention does not require pre-adjustment of particule size.

Inventors measured the particule size of ticagrelor active ingredient with laser diffraction method in their studies. Laser diffraction method is based on the inverse proportion between the particule size and the refraction angle. Laser beams are send onto particules and the beams that are refracted upon colliding with the particules and reflected forward are collected on a dedector after passing through a lens. Beams collected on the dedector are quantified by using a transformer and particule size along with the percentage is calculated by using a computer. Inventors used Malvern Mastersizer 2000 device in their studies to measure particule size of ticagrelor active ingredient. Wet method is preferred where active ingredient particules are dispersed in a dispersion agent (25°C, 2000 rpm, 30 sec.).

Inventors observed that all fomulations have similar solubility profile and content uniformity in their studies where they used formulations of ticagrelor with different particule size. In addition, subject of invention formulation has a better dissolution profile than the Brilanta, current product in the market.

Table 1 shows the comparison of dissolution profiles of the present formulation according to the invention and the product named Brilanta.

Tablo 2 shows the content uniformity data of the formulation according to the invention.

Inventors used ticagrelor active ingredient with D90 value of 15-500 µm in their formulation studies.

D90 value indicates the particule size of the 90 % (volume percent) of the particules.

Excipients of the formulation according to the invention are selected among filler, binder, disintegrants and lubricants.

Filler used in the formulation according to the invention can be selected among mannitol, sorbitol, dextrine, maltodextrine, maltose, laktose, isomalt, pullulane, dibasic calcium phosphate, dibasic calcium phosphate anhydrous, tribasic calcium phosphate, starch, pregelatinized starch, microcrystalline celulose or mixture of these.

The formulation according to the invention contains filler in an amount 20-70%, preferably 30-50% by weight.

Filler used in the formulation according to the invention is preferably selected from mannitol, dibasic calcium phosphate, starch, ni 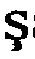 asta, pregelatinized starch or their mixtures.

Binder used in the formulation according to the invention can be selected among hydroxypropyl cellulose, hydroxypropyl methylcellulose, methylcellulose, carboxymethylcellulose, polyvinylpyrrolidone, copovidone, starch, pregelatinized starch, alginic acid, gelatin or their mixtures.

The formulation according to the inention contains binder in an amount of 0-10%, preferably 0-5% by weight.

Binder used in the formulation according to the invention is preferably seleceted from hydroxypropyl cellulose, starch, pregelatinized starch or their mixtures.

Disintegrant used in the formulation according to the invention can be selected among sodium starch glycolate, croscarmellose sodium, crospovidone or their mixtures.

The formulation according to the invention contains disintegrant in an amount of 1-10%, preferably 2-5% by weight.

Disintegrant used in the formulation according to the invention disintegrant is selected from sodium starch glycolate, crospovidon or their mixtures.

Lubricants used in the formulation according to the invention can be selected among stearic acid, magnesium stearate, calcium stearate, polyethylene glycol, sodium stearyl fumarate or their mixtures.

The formulation according to the invention contains lubricants in an amount of 0.5-2% by weight.

Lubricants used in the formulation according to the invention is preferably magnesium stearate.

### Industrial application of the invention

Formulations produced by the production process according to the invention are used in prevention of thrombotic events in patients with acute coronary syndrome.

Below mentioned examples are given just to clarify the subject of invention and can not be used for limitation of the subject of invention in any way.

### Example 1

900 mg ticagrelor active ingredient with a D90 value of 12 µm is dissolved/dispersed in 250 ml ethanol. 1260 mg mannitol, 630 mg dibasic calcium phosphate dihydrate, 90 mg hydroxypropyl cellulose, 90 mg sodium starch glycolate are mixed. Ticagrelor-ethanol solution is added to this mixture and it is kept stirring. Granules obtained are dried, sieved and re-granulated with water. Secondary granules obtained are dried, sieved, mixed with 20 mg magnesium stearate and compressed in tablet form.

### Example 2

900 mg ticagrelor active ingredient with a D90 value of 55 µm is dissolved/dispersed in 250 ml ethanol. 1260 mg mannitol, 630 mg dibasic calcium phosphate dihydrate, 90 mg hydroxypropyl cellulose, 90 mg sodium starch glycolate are mixed. Ticagrelor-ethanol solution is added to this mixture and it is kept stirring. Granules obtained are dried, sieved and re-granulated with water. Secondary granules obtained are dried, sieved, mixed with 20 mg magnesium stearate and compressed in tablet form.

### Example 3

900 mg ticagrelor active ingredient with a D90 value of 142 µm is dissolved/dispersed in 250 ml ethanol. 1260 mg mannitol, 630 mg dibasic calcium phosphate dihydrate, 90 mg hydroxypropyl cellulose, 90 mg sodium starch glycolate are mixed. Ticagrelor-ethanol solution is added to this mixture and it is kept stirring. Granules obtained are dried, sieved and re-granulated with water. Secondary granules obtained are dried, sieved, mixed with 20 mg magnesium stearate and compressed in tablet form.

### Example 4

900 mg ticagrelor active ingredient with a D90 value of 270 µm is dissolved/dispersed in 250 ml ethanol. 1260 mg mannitol, 630 mg dibasic calcium phosphate dihydrate, 90 mg hydroxypropyl cellulose, 90 mg sodium starch glycolate are mixed. Ticagrelor-ethanol solution is added to this mixture and it is kept stirring. Granules obtained are dried, sieved and re-granulated with water. Secondary granules obtained are dried, sieved, mixed with 20 mg magnesium stearate and compressed in tablet form.

### Example 5

900 mg ticagrelor active ingredient with a D90 value of 446 µm is dissolved/dispersed in 250 ml ethanol. 1260 mg mannitol, 630 mg dibasic calcium phosphate dihydrate, 90 mg hydroxypropyl cellulose, 90 mg sodium starch glycolate are mixed. Ticagrelor-ethanol solution is added to this mixture and it is kept stirring. Granules obtained are dried, sieved and re-granulated with water. Secondary granules obtained are dried, sieved, mixed with 20 mg magnesium stearate and compressed in tablet form.

**Table 1 - Comparison of dissolution profiles of the subject of invention formulation and the product Brilanta (distilled water, 0.20 % Tween 80, 75 rpm, 900mL)**

| | **Dissolution** | | | | | |
|---|---|---|---|---|---|---|
| **Time(minutes)** | **Brilanta** | **Example 1** | **Example 2** | **Example 3** | **Example 4** | **Example 5** |
| **5** | **58.50** | **81.70** | **80.90** | **80.50** | **82.10** | **81.50** |
| **10** | **71.40** | **91.80** | **92.00** | **90.80** | **89.80** | **91.60** |
| **20** | **78.90** | **92.10** | **92.50** | **92.30** | **91.00** | **92.10** |
| **30** | **82.60** | **92.90** | **92.80** | **93.10** | **92.80** | **92.70** |

**Table 2 -Content uniformity datas of the formulation obtained by examples according to the invention**

| | **Weight (mg)** | **Measurement Amount** | **Result (%)** | **Average (%)** | **Standart Deviation** |
|---|---|---|---|---|---|
| **Example 1** | 309 | 96.52 | 99.42 | **99.4** | **0.21** |
| **Example 2** | 308.4 | 96.52 | 99.22 | **99.2** | |
| **Example 3** | 307.8 | 96.52 | 99.03 | **99.0** | |
| **Example 4** | 307.7 | 96.52 | 99.00 | **99.0** | |
| **Example 5** | 309.1 | 96.52 | 99.45 | **99.4** | |

## Claims

1. A production process for a formulation containing ticagrelor or a pharmaceutically acceptable salt **characterized in that** ticagrelor or pharmaceutically acceptable salt is dissolved in a solvent or a solvent mixture during production **characterized in that** the process comprising the following steps,
a. preparation of the granulation solution by dissolving ticagrelor active ingredient in a suitable solvent/solvent mixture,
b. dry mixing of one or more excipients,
c. granulation of the dry mixture obtained at step b with the granulation solution containing ticagrelor obtained at step a,
d. drying and sieving granules obtained at step c,
e. re-granulation of the granules obtained at step d with a granulation solvent/solvent mixture different from that used at step a,
f. drying and sieving of the granules obtained at step e and obtaining the desired dosage form.

2. A production process according to the claim 1 **characterized in that** used solvent is selected from methanol, ethanol, n-propanol, isopropanol, n-butanol, isobutanol, dichlomethane, butanone, acetone, dimethyl sulfoxide, glycerine, propylene glycol, glycol, water or mixture of these with each other and/or any pharmaceutically acceptable organic and/or inorganic solvent.

3. A formulation produced according to the production process in claim 1 **characterized in that** the formulation contains ticagrelor or pharmaceutically acceptable salt in an amount of 10-50%, preferably 20-40% by weight.

4. A formulation produced according to the production process in claim 1 **characterized in that** the formulation contains ticagrelor or pharmaceutically acceptable salt in an amount of 30-360 mg, preferably 60-120 mg.

5. A formulation produced according to the production process in claim 1 **characterized in that** the formulation contains at least one excipient selected from filler, binder, disintegrant, lubricant or combination of these.

6. A formulation according to claim 5 **characterized in that** the filler is selected from mannitol, sorbitol, dextrine, maltodextrine, maltose, laktose, isomalt, pullulane, dibasic calcium phosphate, dibasic calcium phosphate anhydrate, tribasic calcium phosphate, starch, pregelatinized starch, microcrystalline celulose or mixture of these.

7. A formulation according to claim 6 **characterized in that** the filler is selected from mannitol, dibasic calcium phosphate, starch, pregelatinized starch or mixture of these.

8. A formulation according to claim 6 and 7 **characterized in that** the formulation contains filler in an amount of 20-70%, preferably 30-50% by weight.

9. A formulation according to claim 5 **characterized in that** the binder is selected from hydroxypropyl cellulose, hydroxypropyl methylcellulose, methylcellulose, carboxymethylcellulose, polyvinylpyrrolidone, copovidone, starch, pregelatinized starch, alginic acid, gelatin or their mixtures.

10. A formulation according to claim 9 **characterized in that** the binder is selected from hydroxypropyl cellulose, starch, pregelatinized starch, alginic acid, gelatin or their mixtures.

11. A formulation according to claim 9 or 10 **characterized in that** the formulation contains binder in an amount of 0-10%, preferably 0-5% by weight.

12. A formulation according to claim 5 **characterized in that** the disintegrant is selected from sodium starch glycolate, croscarmellose sodium, crospovidone or their mixtures.

13. A formulation according to claim 12 **characterized in that** the disintegrant is selecetd from sodium starch glycolate, crospovidone or their mixtures.

14. A formulation according to claim 12 or 13 **characterized in that** the formulation contains disintegrant in an amount of 1-10%, preferably 2-5% by weight.

15. A formulation according to claim 5 **characterized in that** the lubricant is selected from stearic acid, magnesium stearate, calcium stearate, polyethylene glycol, sodium stearyl fumarate or their mixtures.

16. A formulation according to claim 15 **characterized in that** lubricant is magnesium stearate.

17. A formulation according to claim 15 or16 **characterized in that** the formulation contains lubricant in an amount of 0.5-2% by weight.

## Patentansprüche

1. Herstellungsverfahren für eine Formulierung, die Ticagrelor oder ein pharmazeutisch annehmbares Salz enthält, **dadurch gekennzeichnet, dass** Ticagrelor oder ein pharmazeutisch annehmbares Salz während der Herstellung in einem Lösungsmittel oder einem Lösungsmittelgemisch gelöst wird, **dadurch gekennzeichnet, dass** das Verfahren die folgenden Schritte umfasst,
a. Herstellung der Granulationslösung durch Auflösen des Ticagrelor-Wirkstoffs in einem geeigneten Lösungsmittel/Lösungsmittelgemisch,
b. Trockenmischung von einem oder mehreren Hilfsstoffen,
c. Granulierung der in Schritt b erhaltenen Trockenmischung mit der in Schritt a erhaltenen Ticagrelor enthaltenden Granulationslösung,
d. Trocknen und Sieben des in Schritt c erhaltenen Granulats,
e. Re-Granulierung des in Schritt d erhaltenen Granulats mit einem Granulationslösungsmittel/Lösungsmittelgemisch, das sich von dem in Schritt a verwendeten unterscheidet,
f. Trocknen und Sieben des in Schritt e erhaltenen Granulats und Erhalten der gewünschten Dosierungsform.

2. Herstellungsverfahren nach Anspruch 1, **dadurch gekennzeichnet, dass** das verwendete Lösungsmittel ausgewählt ist aus Methanol, Ethanol, n-Propanol, Isopropanol, n-Butanol, Isobutanol, Dichlormethan, Butanon, Aceton, Dimethylsulfoxid, Glycerin, Propylenglykol, Glykol, Wasser oder einer Mischung dieser miteinander und/oder einem beliebigen pharmazeutisch annehmbaren organischen und/oder anorganischen Lösungsmittel.

3. Formulierung, hergestellt gemäß dem Herstellungsverfahren nach Anspruch 1, **dadurch gekennzeichnet, dass** die Formulierung Ticagrelor oder ein pharmazeutisch annehmbares Salz in einer Menge von 10-50 Gew.-%, vorzugsweise 20-40 Gew.-%, enthält.

4. Formulierung, hergestellt gemäß dem Herstellungsverfahren nach Anspruch 1, **dadurch gekennzeichnet, dass** die Formulierung Ticagrelor oder ein pharmazeutisch annehmbares Salz in einer Menge von 30-360 mg, vorzugsweise 60-120 mg, enthält.

5. Formulierung, hergestellt nach dem Herstellungsverfahren in Anspruch 1, **dadurch gekennzeichnet, dass** die Formulierung mindestens einen Hilfsstoff enthält, ausgewählt aus Füllstoff, Bindemittel, Sprengmittel, Gleitmittel oder einer Kombination davon.

6. Formulierung nach Anspruch 5, **dadurch gekennzeichnet, dass** der Füllstoff ausgewählt ist aus Mannitol, Sorbitol, Dextrin, Maltodextrin, Maltose, Laktose, Isomalt, Pullulan, dibasischem Calciumphosphat, dibasischem Calciumphosphatanhydrat, tribasischem Calciumphosphat, Stärke, vorgelatinierter Stärke, mikrokristalliner Cellulose oder einer Mischung davon.

7. Formulierung nach Anspruch 6, **dadurch gekennzeichnet, dass** der Füllstoff ausgewählt ist aus Mannitol, dibasischem Calciumphosphat, Stärke, vorgelatinierter Stärke oder einer Mischung aus diesen.

8. Formulierung nach Anspruch 6 und 7, **dadurch gekennzeichnet, dass** die Formulierung Füllstoff in einer Menge von 20-70 Gew.-%, vorzugsweise 30-50 Gew.-%, enthält.

9. Formulierung nach Anspruch 5, **dadurch gekennzeichnet, dass** das Bindemittel ausgewählt ist aus Hydroxypropylcellulose, Hydroxypropylmethylcellulose, Methylcellulose, Carboxymethylcellulose, Polyvinylpyrrolidon, Copovidon, Stärke, vorgelatinierter Stärke, Alginsäure, Gelatine oder deren Mischungen.

10. Formulierung nach Anspruch 9, **dadurch gekennzeichnet, dass** das Bindemittel ausgewählt ist aus Hydroxypropylcellulose, Stärke, vorgelatinierter Stärke, Alginsäure, Gelatine oder deren Mischungen.

11. Formulierung nach Anspruch 9 oder 10, **dadurch gekennzeichnet, dass** die Formulierung Bindemittel in einer Menge von 0-10 Gew.-%, vorzugsweise 0-5 Gew.-%, enthält.

12. Formulierung nach Anspruch 5, **dadurch gekennzeichnet, dass** das Sprengmittel ausgewählt ist aus Natriumstärkeglycolat, Croscarmellose-Natrium, Crospovidon oder deren Mischungen.

13. Formulierung nach Anspruch 12, **dadurch gekennzeichnet, dass** das Sprengmittel ausgewählt ist aus Natriumstärkeglycolat, Crospovidon oder deren Mischungen.

14. Formulierung nach Anspruch 12 oder 13, **dadurch gekennzeichnet, dass** die Formulierung Sprengmittel in einer Menge von 1-10 Gew.-%, vorzugsweise 2-5 Gew.-%, enthält.

15. Formulierung nach Anspruch 5, **dadurch gekennzeichnet, dass** das Gleitmittel ausgewählt ist aus Stearinsäure, Magnesiumstearat, Calciumstearat, Polyethylenglykol, Natriumstearylfumarat oder deren Mischungen.

16. Formulierung nach Anspruch 15, **dadurch gekennzeichnet, dass** das Gleitmittel Magnesiumstearat ist.

17. Formulierung nach Anspruch 15 oder 16, **dadurch gekennzeichnet, dass** die Formulierung Gleitmittel in einer Menge von 0.5-2 Gew.-% enthält.

## Revendications

1. Procédé de production d'une formulation contenant du ticagrélor ou un sel pharmaceutiquement acceptable, **caractérisé en ce que** le ticagrélor ou un sel pharmaceutiquement acceptable est dissous dans un solvant ou un mélange de solvants au cours de la production, **caractérisé en ce que** le procédé comprend les étapes suivantes,
a. préparation de la solution de granulation par dissolution de l'ingrédient actif ticagrélor dans un mélange solvant/solvant approprié,
b. mélange à sec d'un ou plusieurs excipients,
c. granulation du mélange sec obtenu à l'étape b avec la solution de granulation contenant du ticagrélor obtenue à l'étape a,
d. séchage et tamisage des granules obtenus à l'étape c,
e. re-granulation des granules obtenus à l'étape d avec un mélange solvant/solvant de granulation différent de celui utilisé à l'étape a,
f. séchage et tamisage des granules obtenus à l'étape e et obtention de la forme de dosage souhaitée.

2. Un procédé de production selon la revendication 1, **caractérisé en ce que** le solvant utilisé est choisi parmi le méthanol, l'éthanol, le n-propanol, l'isopropanol, le n-butanol, l'isobutanol, le dichlorométhane, la butanone, l'acétone, le diméthyl sulfoxyde, la glycérine, le propylène glycol, le glycol, l'eau ou mélange de ceux-ci les uns avec les autres et/ou tout solvant organique et/ou inorganique pharmaceutiquement acceptable.

3. Une formulation produite selon le procédé de production selon la revendication 1, **caractérisée en ce que** la formulation contient du ticagrélor ou un sel pharmaceutiquement acceptable en une quantité de 10-50%, de préférence de 20-40% en poids.

4. Une formulation produite selon le procédé de production selon la revendication 1, **caractérisée en ce que** la formulation contient du ticagrélor ou un sel pharmaceutiquement acceptable en une quantité de 30-360 mg, de préférence de 60-120 mg.

5. Une formulation produite selon le procédé de production selon la revendication 1, **caractérisée en ce que** la formulation contient au moins un excipient choisi parmi une charge, un liant, un désintégrant, un lubrifiant ou une combinaison de ceux-ci.

6. Une formulation selon la revendication 5 **caractérisée en ce que** la charge est choisie parmi le mannitol, le sorbitol, la dextrine, la maltodextrine, le maltose, le lactose, l'isomalt, le pullulane, le phosphate de calcium dibasique, le phosphate de calcium dibasique anhydraté, le phosphate de calcium tribasique, l'amidon, l'amidon prégélatinisé, la cellulose microcristalline ou un mélange de ceux-ci.

7. Une formulation selon la revendication 6, **caractérisée en ce que** la charge est choisie parmi le mannitol, le phosphate de calcium dibasique, l'amidon, l'amidon prégélatinisé ou un mélange de ceux-ci.

8. Une formulation selon la revendication 6 et 7, **caractérisée en ce que** la formulation contient une charge en une quantité de 20-70%, de préférence de 30-50% en poids.

9. Une formulation selon la revendication 5 **caractérisée en ce que** le liant est choisi parmi l'hydroxypropyl cellulose, l'hydroxypropyl méthylcellulose, la méthylcellulose, la carboxyméthylcellulose, la polyvinylpyrrolidone, la copovidone, l'amidon, l'amidon prégélatinisé, l'acide alginique, la gélatine ou leurs mélanges.

10. Une formulation selon la revendication 9 **caractérisée en ce que** le liant est choisi parmi l'hydroxypropyl cellulose, l'amidon, l'amidon prégélatinisé, l'acide alginique, la gélatine ou leurs mélanges.

11. Une formulation selon la revendication 9 ou 10, **caractérisée en ce que** la formulation contient un liant en une quantité de 0-10%, de préférence de 0-5% en poids.

12. Une formulation selon la revendication 5, **caractérisée en ce que** le désintégrant est choisi parmi le glycolate d'amidon sodique, la croscarmellose sodique, la crospovidone ou leurs mélanges.

13. Une formulation selon la revendication 12, **caractérisée en ce que** le désintégrant est choisi parmi le glycolate d'amidon sodique, la crospovidone ou leurs mélanges.

14. Une formulation selon la revendication 12 ou 13, **caractérisée en ce que** la formulation contient un désintégrant en une quantité de 1-10%, de préférence de 2-5% en poids.

15. Une formulation selon la revendication 5, **caractérisée en ce que** le lubrifiant est choisi parmi l'acide stéarique, le stéarate de magnésium, le stéarate de calcium, le polyéthylène glycol, le stéaryl fumarate de sodium ou leurs mélanges.

16. Une formulation selon la revendication 15, **caractérisée en ce que** le lubrifiant est le stéarate de magnésium.

17. Une formulation selon la revendication 15 ou 16, **caractérisée en ce que** la formulation contient un lubrifiant en une quantité de 0.5-2% en poids.
